# EUROPÄISCHE PATENTSCHRIFT

(11) **EP 0 743 849 B1**
(45) Veröffentlichungstag und Bekanntmachung des Hinweises auf die Patenterteilung: **08.11.2000**
(21) Anmeldenummer: 95908263.7
(22) Anmeldetag: 04.02.1995
(51) Int. Cl.: A61K 7/13

(54) **VERWENDUNG VON 1,2,3,4-TETRAHYDROCHINOXALINEN ALS OXIDATIONSFARBSTOFFVORPRODUKTE IN OXIDATIONSFÄRBEMITTELN**
USE OF 1,2,3,4-TETRAHYDROQUINOXALINES AS OXIDATION DYE PRECURSORS IN OXIDATION DYES
UTILISATION DE 1,2,3,4-TETRAHYDROQUINOXALINES COMME PRECURSEURS DE COLORANTS PAR OXYDATION DANS DES TEINTURES PAR OXYDATION

(30) Priorität: 12.02.1994 DE 4404564
(43) Veröffentlichungstag der Anmeldung: 27.11.1996
(73) Patentinhaber: Henkel Kommanditgesellschaft auf Aktien, 40191 Düsseldorf (DE)
(72) Erfinder: KNÜBEL, Georg, D-40229 Düsseldorf (DE); ROSE, David, D-40723 Hilden (DE); HÖFFKES, Horst, D-40595 Düsseldorf (DE); MEINIGKE, Bernd, D-51371 Leverkusen (DE)
(74) Vertreter: Wilk, Hans-Christof, Dr.
(86) Internationale Anmeldenummer: EP9500404
(87) Internationale Veröffentlichungsnummer: WO9521604

(56) Entgegenhaltungen:
- CH-A- 619 476
- DE-A- 1 956 158
- DE-B- 1 804 066

## Beschreibung

Die Erfindung betrifft die Verwendung von 1,2,3,4-Tetrahydrochinoxalinen als Oxidationsfarbstoffvorprodukte in Oxidationsfärbemitteln zum Färben von Keratinfasern, insbesondere menschlichen Haaren. Die 1,2,3,4-Tetrahydrochinoxaline eignen sich besonders als Kupplerkomponenten gemeinsam mit Entwicklerkomponenten.

Oxidationsfärbemittel enthalten üblicherweise Oxidationsfarbstoffvorprodukte in einem wäßrigen Träger; als Oxidationsfarbstoffvorprodukte werden Entwicklerkomponenten und Kupplerkomponenten eingesetzt. Die Entwicklerkomponenten bilden unter Einfluß von Oxidationsmitteln oder von Luftsauerstoff untereinander oder unter Kupplung mit einer oder mehreren Kupplerkomponenten die eigentlichen Farbstoffe aus.

Die deutsche Auslegeschrift 18 04 066 offenbart leicht abziehbare Haarfärbemittel auf Basis bekannter Kupplungskomponenten und heterocyclischen Aminoverbindungen, z. B. 6-Amino-1,2,3,4-Tetrahydrochinoxalin.

Die Verwendung von nitro-substituierten 1,2,3,4-Nitrochinoxalinen als direktziehende Haarfarbstoffe ist aus den deutschen Offenlegungsschriften DE-A-3825 212 und DE-A-42 06 537 bekannt. Es wurde nun gefunden, daß spezifisch substituierte 1,2,3,4-Tetrahydrochinoxaline sich in besondere. Maße als Oxidationsfarbstoffvorprodukte in Oxidationsfärbemitteln für Keratinfasern eignen, da sie die an Oxidationsfärbemittelvorprodukte zu stellenden Anforderungen (hohe Farbintensität der erzielten Ausfärbungen, Lichtechtheit, Waschechtheit, Reibechtheit) in besonderem Maße erfüllen. Sie fungieren dabei als Kupplerkomponenten und unterscheiden sich von konventionellen Kupplern durch ihre ortho-Diamino-Funktionseinheit; üblicherweise sind Kupplerkomponenten meta-disubstituierte Aromaten.

Gegenstand der Erfindung ist die Verwendung von 1,2,3,4-Tetrahydrochinoxalinen der Formel I 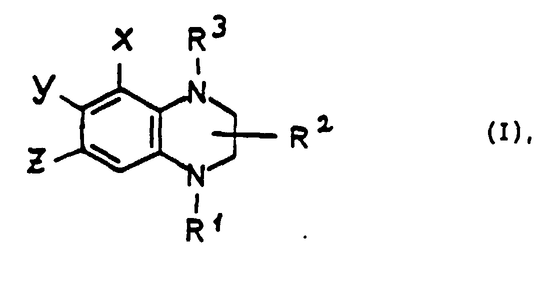 in der R¹, R² und R³ unabhängig voneinander Wasserstoffe, C₁-C₄-Alkylgruppen, Benzylgruppen, 2-Phenylethylgruppen oder C₂-C₄-Hydroxyalkylgruppen bedeuten und X, Y und Z für Wasserstoffe, Fluor-, Chlor- Brom- oder Jodatome oder C₁-C₄-Alkylgruppen stehen, wobei jedoch mindestens eine der Gruppen X, Y und Z für Wasserstoff steht, als Oxidationsfarbstoffvorprodukte in Oxidationsfärbemitteln zum Färben von Keratinfasern. Unter Keratinfasern sind Pelze, Wolle oder Federn zu verstehen, insbesondere jedoch menschliche Haare.

Besonders bevorzugt sind 1,2,3,4-Tetrahydrochinoxaline der Formel I, in denen R² und Z Wasserstoffe bedeuten, R¹ und R³ für Wasserstoffe oder C₂-C₄-Hydroxyalkylgruppen stehen und X und Y Methylgruppen oder Chloratome darstellen. Insbesondere sind 5-Methyl- und 6-Methyl-1,2,3,4-tetrahydrochinoxalin bevorzugt.

Die Erfindung schließt die Salze der 1,2,3,4-Tetrahydrochinoxaline der Formel I mit ein; insbesondere sind die Hydrochloride, Hydrobromide und Sulfate zu nennen.

Einige der 1,2,3,4-Tetrahydrochinoxaline der Formel I sind literaturbekannte Verbindungen, die sich nach dem Muster der im Beispielteil zitierten Literaturstellen synthetisieren lassen. Andere wiederum stellen neue Substanzen dar, die sich aus den entsprechenden Chinoxalinen oder Nitrochinoxalinen durch katalytische Hydrierung herstellen lassen; eine nähere Beschreibung erfolgt im Beispielteil.

Ein weiterer Erfindungsgegenstand sind demnach neue 1,2,3,4-Tetrahydrochinoxaline der Formel I, wobei mindestens eine der Gruppe R¹ und R³ für eine C₂ - C₄ - Hydroxyalkylgruppe steht.

Ein weiterer Erfindungsgenstand sind neue 1,2,3,4-Tetrahydrochinoxaline der Formel I, wobei X und Y gleichzeitig C₁-C₄-Alkylgruppen darstellen.

Die 1,2,3,4-Tetrahydrochinoxaline der Formel I liefern mit üblichen Entwicklerkomponenten ein breites Spektrum anwendungstechnisch interessanter Oxidationsfarben im Bereich gelber bis dunkelbrauner Nuancen, die sich wegen ihrer Brillanz und guten Echtheitseigenschaften insbesondere zum Färben von menschlichen Haaren eignen.

Übliche Entwicklerkomponenten sind z.B. primäre aromatische Amine mit einer weiteren in para- oder ortho-Position befindlichen freien oder substituierten Hydroxy- oder Aminogruppe, Diaminopyridin-Derivate, heterocyclische Hydrazone, 4-Aminopyrazolon-Derivate, 2,4,-5,6-Tetraaminopyrimidin, 2,4,5-Triamino-6-hydroxy-pyrimidin, 5,6-Diamino-2,4-dihydroxypyrimidin und deren Derivate. Einige speziell geeignete Entwicklerkomponenten sind im Beispielteil aufgeführt.

Ein weiterer Patentgegenstand sind deshalb Oxidationsfärbemittel zum Färben von menschlichen Haaren enthaltend Oxidationsfarbstoffvorprodukte in einem wasserhaltigen Träger, wobei die 1,2,3,4-Tetrahydrochinoxaline der Formel I als Kuppler in einer Menge von 0,05 bis 20 mMol sowie übliche Entwicklerkomponenten in einer Menge von 0,05 bis 20 mMol und gegebenenfalls übliche direktziehende Farbstoffe in einer Menge von 0,05 bis 20 mMol, jeweils pro 100 g des Färbemittels, enthalten sind.

Die erfindungsgemäßen Haarfärbemittel können neben den 1,2,3,4-Tetrahydrochinoxalinen der Formel I auch andere bekannte Kupplerkomponenten enthalten, die zur Modifizierung der Farbnuancen und zur Erzeugung natürlicher Farbtöne erforderlich sind. Solche üblichen Kupplerverbindungen sind z.B. meta-Phenylendiaminderivate, Naphthole, Resorcinderivate und Pyrazolone. Gegebenenfalls können auch mehrere bekannte Entwicklerkomponenten und direktziehende Farbstoffe zur weiteren Modifizierung der Farbnuancen eingesetzt werden. Geeignete direktziehende Farbstoffe sind z.B. Nitrophenylendiamine, Nitroaminophenole, Anthrachinonfarbstoffe oder Indophenole.

Es ist auch nicht erforderlich, daß die 1,2,3,4-Tetrahydrochinoxaline der Formel I einheitliche Verbindungen sind. Vielmehr können auch Gemische verschiedener Verbindungen der Formel I verwendet werden.

In den erfindungsgemäßen Haarfärbemitteln werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können. Zur Herstellung der erfindungsgemäßen Färbemittel werden die Oxidationsfarbstoffvorprodukte in einem geeigneten wasserhaltigen Träger eingearbeitet. Solche Träger sind z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Der wasserhaltige Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z. B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Die Bestandteile des wasserhaltigen Trägers werden zur Herstellung der erfindungsgemäßen Haarfärbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht ist. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Machwaschen mit einem Shampoo entfällt, wenn ein stark tensidhaltiger Träger, z.B. ein Färbeshampoo verwendet wurde.

Die nachfolgenden Beispiele sollen den Erfindungsgegenstand näher erläutern, ohne ihn jedoch hierauf zu beschränken.

### Beispiele

### I. Herstellungsbeispiele

**a. Ausgewählte literaturbekakannte 1,2,3,4-Tetrahydrochinoxialine**
   1) 1,2,3,4-Tetrahydrochinoxalin (Beilstein E V 23/6, 24; E III/IV 23/6, 980; E II 23. 102; H 23, 106)
   2) 6-Methyl-1,2,3,4-tetrahydrochinoxalin (Beilstein E V 23/6, 59; E III/IV 23/6, 977; E II 23, 107)
   3) 6-Chlor-1,2,3,4-tetrahydrochinoxalin (Beilstein E V 23/6, 27; E III/IV 23/6, 985)
**b. Synthese neuer 1,2,3,4-Tetrahydrochinoxaline**
   4) 5,6-Dimethyl-1,2,3,4-Tetrahydrochinoxalin
      5.3 g (0,034 mol) 5,6-Dimethylchinoxalin wurden in 200 ml Ethanol gelöst und nach Zugabe von 1 g Raney-Nickel 2 h bei Raumtemperatur und Normaldruck hydriert. Nach Filtration wurde bis zur Trockne eingeengt und aus Methyl-t-butylether umkristallisiert.

Ausbeute: 3.2 g (59 %)
Smp.: 72 - 73°C

### II. Anwendungsbeispiele

Es wurde ein erfindungsgemäßes Färbemittel in Form einer Haarfärbecreme-Emulsion der folgenden Zusammensetzung hergestellt

| | |
|---|---|
| Fettalkohol C_{12/18} | 10,0 g |
| Fettalkohol C_{12/14} + 2 E0-sulfat (Na-salz, 28 %ig) | 25,0 g |
| Wasser | 60,0 g |
| 1,2,3,4-Tetrahydrochinoxalin der Formel I gemäß Beispiel 1 - 4 | 6,5 mMol |
| Entwicklerkomponente (PTD, PAP oder TAP) | 6,5 mMol |
| Ammoniumsulfat | 1,0 g |
| konz. Ammoniaklösung bis pH = | 9,5 |
| Wasser | ad 100 g |
| PTD: para-Toluylendiamin PAP: para-Aminophenol TAP: Tetraaminopyrimidin | |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 9,5 eingestellt, dann wurde mit Wasser auf 100 g aufgefüllt.

Die oxidative Entwicklung der Färbung wurde entweder durch Luftoxidation oder mit Hilfe 3 %iger Wasserstoffperoxidlösung als Oxidationsmittel durchgeführt. Hierzu wurden 100 g der Emulsion mit 50 g Wasserstoffperoxidlösung versetzt und vermischt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Ausfärbungen von PTD, PAP und TAP mit 1,2,3,4-Tetrahydrochinoxalinen
1) 1,2,3,4-Tetrahydrochinoxalin:
   - PTD:: oliv
   - PAP:: olivbraun
   - TAP:: maisgelb
2) 6-Methyl-1,2,3,4-tetrahydrochinoxalin:
   - PTD:: dunkelgelb
   - PAP:: linoleumbraun
   - TAP:: gelbbraun
3) 6-Chlor-1,2,3,4-tetrahydrochinoxalin:
   - PTD:: kaffeebraun
   - TAP:: korngelb
4) 5,6-Dimethyl-1,2,3,4-tetrahydrochinoxalin:
   - PTD:: braungelb
   - PAP:: curry
   - TAP:: korngelb

## Patentansprüche

1. Verwendung von 1,2,3,4-Tetrahydrochinoxalinen der Formel I 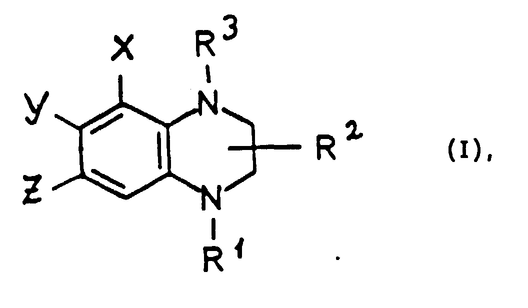 in der R¹, R² und R³ unabhängig voneinander Wasserstoffe, C₁-C₄--Alkylgruppen, Benzylgruppen, 2-Phenylethylgruppen oder C₂-C₄-Hydroxyalkylgruppen bedeuten und X, Y und Z für Wasserstoffe, Fluor-, Chlor-, Brom- oder Jodatome oder C₁-C₄-Alkylgruppen stehen, wobei jedoch mindestens eine der Gruppen X, Y und Z für Wasserstoff steht, als Oxidationsfarbstoffvorprodukte in Oxidationsfärbemitteln zum Färben von Keratinfasern.

2. Verwendung nach Anspruch 1, dadurch gekennzeichnet, daß in den 1,2,3,4-Tetrahydrochinoxalinen der Formel I R² und Z Wasserstoffe bedeuten, R¹ und R³ für Wasserstoffe oder C₂-C₄-Hydroxyalkylgruppen stehen und X und Y Methylgruppen oder Chloratome darstellen.

3. Verwendung nach Anspruch 1 und 2, dadurch gekennzeichnet, daß das 1,2,3,4-Tetrahydrochinoxalin der Formel I 5-Methyl- oder 6-Methyl-1,2,3,4-tetrahydrohinoxalin ist.

4. 1,2,3,4-Tetrahydrochinoxaline der Formel I gemäß Anspruch 1, wobei mindestens eine der Gruppen R¹ und R³ für eine C₂-C₄-Hydroxyalkylgruppe steht.

5. 1,2,3,4-Tetrahydrochinoxaline der Formel I gemäß Anspruch 1, wobei X und Y gleichzeitig C₁-C₄-Alkylgruppen darstellen.

6. Oxidationsfärbemittel zum Färben von menschlichen Haaren, enthaltend Oxidationsfarbstoffvorprodukte in einem wasserhaltigen Träger, dadurch gekennzeichnet, daß 1,2,3,4-Tetrahydrochinoxaline der Formel I als Kuppler in einer Menge von 0,05 bis 20 mMol sowie übliche Entwicklerkomponenten in einer Menge von 0,05 bis 20 mMol und gegebenenfalls übliche direktziehende Farbstoffe in einer Menge von 0,05 bis 20 mMol, jeweils pro 100 g des Färbemittels, enthalten sind.

## Claims

1. The use of 1,2,3,4-tetrahydroquinoxalines corresponding to formula I: 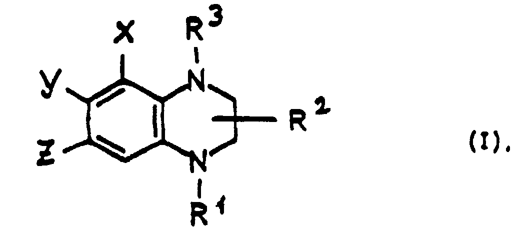 in which R¹, R² and R³ independently of one another represent hydrogen, C₁₋₄ alkyl groups, benzyl groups, 2-phenylethyl groups or C₂₋₄ hydroxyalkyl groups and X, Y and Z represent hydrogen, fluorine, chlorine, bromine or iodine atoms or C₁₋₄ alkyl groups, although at least one of the groups X, Y and Z is hydrogen,
as oxidation dye precursors in oxidative coloring formulations for coloring keratin fibers.

2. The use claimed in claim 1, characterized in that, in the 1,2,3,4-tetrahydroquinoxalines corresponding to formula I, R² and Z are hydrogen, R¹ and R³ are hydrogen or C₂₋₄ hydroxyalkyl groups and X and Y are methyl groups or chlorine atoms.

3. The use claimed in claims 1 and 2, characterized in that the 1,2,3,4-tetrahydroquinoxaline corresponding to formula I is 5-methyl- or 6-methyl-1,2,3,4-tetrahydroquinoxaline.

4. 1,2,3,4-Tetrahydroquinoxalines corresponding to formula I in claim 1, in which at least one of the groups R¹ and R³ is a C₂₋₄ hydroxyalkyl group.

5. 1,2,3,4-Tetrahydroquinoxalines corresponding to formula I in claim 1, in which X and Y are both C₁₋₄ alkyl groups.

6. Oxidative coloring formulations for coloring human hair containing oxidation dye precursors in a water-containing carrier, characterized in that 1,2,3,4-tetrahydroquinoxalines corresponding to formula I are present as secondary intermediates in a quantity of 0.05 to 20 mmoles, typical primary intermediates are present in a quantity of 0.05 to 20 mmoles and, optionally, typical substantive dyes are present in a quantity of 0.05 to 20 mmoles per 100 g of the coloring formulation.

## Revendications

1. Utilisation de 1,2,3,4-tétrahydroquinoxalines de la formule I 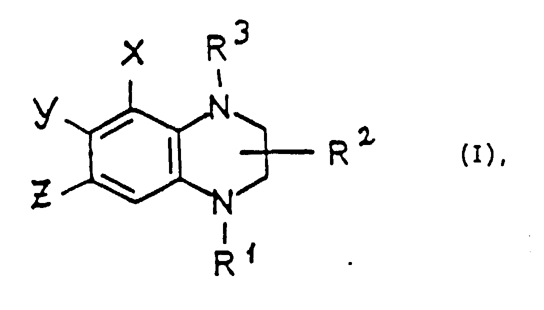 dans laquelle R¹, R² et R³ représentent indépendamment les uns des autres l'hydrogène, un groupe alkyle en C₁-C₄, un groupe benzyle, un groupe 2-phényléthyle ou un groupe hydroxyalkyle en C₂-C₄, et X, Y et Z correspondent à l'hydrogène, à un atome de fluor, de chlore, de brome ou d'iode ou à un groupe alkyle en C₁-C₄, au moins un des groupes X, Y et Z correspondant toutefois à l'hydrogène, comme précurseurs de colorants d'oxydation dans des teintures d'oxydation pour la coloration de fibres kératiniques.

2. Utilisation selon la revendication 1, caractérisée en ce que dans les 1,2,3,4-tétrahydroquinoxalines de la formule I, R² et Z représentent l'hydrogène, R¹ et R³ correspondent à l'hydrogène ou à un groupe hydroxyalkyle en C₂-C₄, et X et Y sont un groupe méthyle ou un atome de chlore.

3. Utilisation selon les revendications 1 et 2, caractérisée en ce que la 1,2,3,4-tétrahydroquinoxaline de la formule I est une 5-méthyl- ou une 6-méthyl-1,2,3,4-tétrahydroquinoxaline.

4. 1,2,3,4-tétrahydroquinoxalines de la formule I selon la revendication 1, dans lesquelles au moins un des groupes R¹ et R³ représente un groupe hydroxyalkyle en C₂-C₄.

5. 1,2,3,4-tétrahydroquinoxalines de la formule I selon la revendication 1, dans lesquelles X et Y représentent simultanément un groupe alkyle en C₁-C₄.

6. Teintures d'oxydation pour la coloration des cheveux humains, contenant des précurseurs de colorants d'oxydation dans un vecteur aqueux, caractérisées en ce que les 1,2,3,4-tétrahydroquinoxalines de la formule I sont contenues comme copulateurs dans une proportion de 0,05 à 20 mmol conjointement avec des composants développateurs usuels dans une proportion de 0,05 à 20 mmol et, éventuellement, avec des colorants montant directement sur la fibre usuels dans une proportion de 0,05 à 20 mmol, dans chaque cas pour 100 g de la teinture.
